(19) Europäisches Patentamt
European Patent Office
Office européen des brevets

(11) Publication number: **0 369 686 A1**

(12) # EUROPEAN PATENT APPLICATION

(21) Application number: **89311636.8**

(51) Int. Cl.[5]: **C07D 463/00, A61K 31/435**

(22) Date of filing: **10.11.89**

(30) Priority: **14.11.88 US 271794**

(43) Date of publication of application:
**23.05.90 Bulletin 90/21**

(84) Designated Contracting States:
**AT BE CH DE ES FR GB GR IT LI LU NL SE**

(71) Applicant: **ELI LILLY AND COMPANY**
**Lilly Corporate Center**
**Indianapolis Indiana 46285(US)**

(72) Inventor: **Eckrich, Thomas Michael**
**6534 St. James Drive**
**Indianapolis Indiana 46217(US)**
Inventor: **Pasini, Carol Elaine**
**1017 New Amsterdam Drive**
**Greenwood Indiana 46142(US)**

(74) Representative: **Tapping, Kenneth George et al**
**Lilly Industries Limited Patent Department**
**Erl Wood Manor**
**Windlesham Surrey, GU20 6PH(GB)**

(54) **Hydrates of beta-lactam antibiotic.**

(57) The crystalline dihydrate and trihydrate forms of the antibiotic 7β-[2′-(R)-2′-phenyl-2′-aminoacetamido]-3-chloro-3-(1-carbadethiacephem)-4-carboxylic acid ("LY163892") are described. These hydrates are antibiotics in their own right, and are valuable intermediates to the corresponding monohydrate form of LY163892.

EP 0 369 686 A1

## HYDRATES OF β-LACTAM ANTIBIOTIC

This invention relates to novel hydrate forms of a β-lactam antibiotic, more particularly to novel crystalline dihydrate and trihydrate forms of a 1-carbacephalosporin.

The β-lactam antibiotic of the Formula I

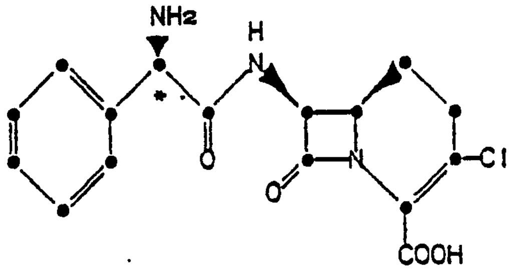

I

is a potent orally-active antibiotic. The antibiotic is described, for example, by J. Hashimoto et al. in U.S. Patent No. 4,335,211, issued June 15, 1982. For brevity's sake, the compound of Formula I will be referred to by the number LY163892.

The instant invention is directed to the crystalline dihydrate and trihydrate of LY163892. These two hydrates are pharmaceutically elegant hydrates of the parent compound.

In addition, LY163892 dihydrate and trihydrate are convenient intermediates in the synthesis of LYI63892 monohydrate. LY163892 monohydrate is a pharmaceutically elegant form of the parent compound possessing excellent stability and bulk handling qualities over a wide range of humidity and other manufacturing conditions. LY163892 monohydrate is further described in EP-A-311366.

One aspect of the present invention is directed to the crystalline dihydrate of LY163892, whose formula is given below as Formula I. More specifically, the invention is directed to LY163892 dihydrate having the X-ray powder diffraction pattern listed below in Table I. Further aspects of the invention are pharmaceutical formulations of crystalline LY163892 dihydrate.

Yet another aspect of the instant invention is the crystalline trihydrate of LY163892. A preferred embodiment of this second aspect is a crystalline trihydrate possessing the X-ray powder diffraction pattern of Table II below. The invention also encompasses pharmaceutical formulations of the crystalline trihydrate.

The instant invention is directed to the crystalline dihydrate and trihydrate of the antibiotic compound of Formula I:

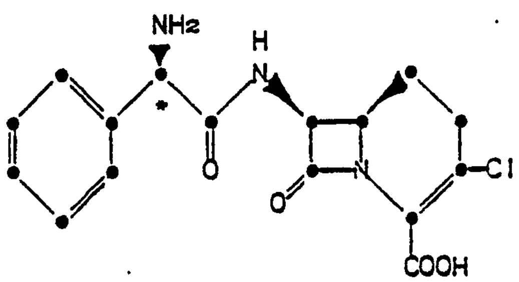

I

The 2′ position carbon of the antibiotic in Formula I exists as the "R" absolute configuration. Furthermore, the compound of Formula I can also be in the form of a zwitterion, a form which is also encompassed in the various aspects of the instant invention. For brevity's sake, the compound of Formula I will be referred to as "LY163892", and the compounds of the instant invention as either "LY163892 dihydrate" or "LY163892 trihydrate" or, more simply, the "dihydrate" or the "trihydrate", respectively.

The dihydrate is a white microcrystalline solid. A preferred embodiment of this aspect of the invention is the crystalline dihydrate characterized by the X-ray powder diffraction pattern of Table I:

Table I

| Dihydrate Crystal Pattern | |
|---|---|
| d | $I/I_1$ |
| 16.35 | 1.00 |
| 9.82 | .05 |
| 9.50 | .05 |
| 8.18 | .90 |
| 6.46 | .01 |
| 6.32 | .04 |
| 5.40 | .02 |
| 4.72 | .11 |
| 4.48 | .04 |
| 4.07 | .19 |
| 3.88 | .02 |
| 3.78 | .07 |
| 3.60 | .08 |
| 3.45 | .04 |
| 3.31 | .01 |
| 3.24 | .05 |
| 3.10 | .02 |
| 2.99 | .05 |
| 2.92 | .05 |

The diffraction pattern in Table I was obtained with nickel-filtered copper radiation (Cu:Ni) of wavelength $\lambda$ = 1.5418 Å. The interplanar spacings are in the column marked "d" and are in Angstroms and the relative intensities are in the column marked "$I/I_1$".

Further aspects of the present invention are pharmaceutical formulations of LY163892 dihydrate. Preferred embodiments of the invention include pharmaceutical formulations containing the dihydrate crystalline form of LY163892 characterized by the crystal pattern of Table I.

LY163892 trihydrate is also a white microcrystalline solid. A preferred embodiment of this aspect of the invention is the crystalline trihydrate characterized by the X-ray powder diffraction pattern of Table II:

Table II

| Trihydrate Crystal Pattern | |
|---|---|
| d | $I/I_1$ |
| 11.94 | 1.00 |
| 7.94 | .15 |
| 5.94 | .05 |
| 5.27 | .03 |
| 4.67 | .02 |
| 3.95 | .18 |
| 3.63 | .03 |
| 3.34 | .03 |
| 3.07 | .03 |

The diffraction pattern in Table II was obtained using the same parameters as those employed to obtain the

pattern set forth in Table I above.

A further aspect of the invention includes pharmaceutical formulations of LY163892 trihydrate. Preferred embodiments of this aspect of the invention include pharmaceutical formulations containing the trihydrate crystalline form of LY163892 characterized by the crystal pattern of Table II.

The dihydrate and trihydrate can be made from the various forms of LY163892 such as the anhydrate, the DMF solvates, or the various combinations of DMF solvate - hydrate. The synthesis of certain of such starting materials is discussed below in the Experimental Section. The preferred starting material for the dihydrate is the mono(DMF) solvate, while the preferred starting material for the trihydrate is the bis(DMF) solvate, anhydrate or the ethanol solvate of LY163892.

In preparing the dihydrate, the DMF solvate starting material (for example) is suspended in water. Solution is effected by acidifying the suspension, for example, by the addition of concentrated hydrochloric acid until a solution results. The acidified solution can be stabilized by the addition of one or more equivalents (versus the starting material) of sodium EDTA. The solution is further acidified, to give an approximate pH of 0.5 to 1.5 at a temperature of about 5° C to 50° C, preferably 10°-15° C, optionally seeded with the dihydrate of LY163892, stirred to induce crystallization (usually about thirty minutes), then the pH is adjusted upward to about 3.5 to 4.0 by the addition of base (such as tri-ethylamine) to complete crystallization. The crystals of dihydrate are collected by conventional filtration techniques and allowed to dry in the open at room tempera-ture. Alternatively, the filter cake may be stirred with water, refiltered, and collected and washed with acetonitrile. After drying in the open, the crystalline dihydrate is obtained.

The trihydrate is made by a procedure similar to the dihydrate. A LY163892 DMF solvate (for example), preferably the bis(DMF) solvate, is suspended in water, the suspension is warmed to about 50° C, and the suspension is acidified (for example, to pH 1.57) with concentrated hydrochloric acid and stirred to effect solution (typically about thirty minutes). A stabilizer such as sodium EDTA may be added. The solution is filtered, and the filtrate is first seeded with crystalline LY163892 monohydrate and acidified (for example, to pH 2.1 with concentrated hydrochloric acid) to induce crystallization. The resultant suspension is stirred (typically thirty minutes) and the suspension is treated with a base such as triethylamine to take the pH to the isoelectric point (approximately pH 4.8-4.9) of the monohydrate, thus enhancing the yield of crystalline monohydrate. After filtering to collect the crude monohydrate thus formed, the filtrate is mixed with an equal volume of acetonitrile. The (developing) suspension is stirred for one hour and filtered. The filter cake is washed with acetonitrile and air dried to give the crystalline trihydrate. Alternatively, the (preferably) bis-(DMF) solvate is suspended in water, stabilized with sodium EDTA and the solution warmed to approximately 50° C. The pH of the suspension is raised with, for example, aqueous saturated sodium bicarbonate solution (to approximately 8.6) to effect solution. The solution is stirred and neutralized (approximate pH 7.6) to induce crystallization. The suspension is acidified to the isoelectric point (about pH 4.6) typically with concentrated hydrochloric acid, to complete crystallization. The crystals are collected by filtration and air dried to give crystalline trihydrate.

As noted above, LY163892 dihydrate and LY163892 trihydrate are also useful as intermediates to LY163892 monohydrate. The monohydrate is prepared by first suspending either of the above starting materials in water. The most common procedure is to effect solution of the starting material by the addition of a minimum amount of acid, generally 6N (or more dilute) hydrochloric acid. Alternatively, a solution of the starting material is effected by adding the minimum amount of base (for example, the minimum amount of 2N sodium hydroxide, resulting in a pH of about 7.6).

Regardless of how solution is effected, crystallization is induced by slowly adjusting the pH of the solution of starting material to approximately 4, and preferably 4.8. For example, if the solution is effected by the addition of acid, it is preferred to raise the temperature of the solution to about 50° C and slowly add triethylamine (alternatively, sodium hydroxide) to the solution until a pH of approximately 4.8 is obtained. The gradually developing suspension is stirred and maintained at about 50° C during the addition of the base. Seeding the solution with a small amount of crystalline monohydrate early in the addition period of base is preferred. For example, seeding is often done when the pH of the solution is approximately 1.8. If the starting material solution was effected by the addition of base, the pH is slowly taken to approximately 4 by the addition of an acid (preferably 2N hydrochloric acid). Effecting solution of the starting material with hydrochloric acid and inducing crystallization by adjusting the pH of the solution to approximately 4.8 with triethylamine is preferred.

The suspension resulting from adjusting the pH of starting material solution to approximately 4 is isolated by conventional filtering techniques, such as vacuum filtration on a Büchner funnel. The collected crystals are washed and allowed to dry in air at ambient temperature. Alternatively, the warm pH-adjusted suspensions (50° C) are cooled to approximately 20° C, stirred, filtered (such as on a Büchner funnel) and the collected solid dried at 30° C for 24 to 48 hours by conventional means (such as a clean-air oven).

As stated above, a further aspect of this invention is the pharmaceutical compositions of the antibiotic compounds of the dihydrate and trihydrate of Formula I. In particular, these pharmaceutical compositions are useful for the control of gram-positive and gram-negative bacterial infections in warm-blooded animals and comprise a suitable vehicle and a therapeutically effective amount of the antibiotic compounds of the dihydrate and trihydrate of Formula I.

With regard to compositions for oral administration such as tablets and capsules, the term "suitable vehicle" means common excipients such as binding agents, for example, syrup, acacia, gelatin, sorbitol, tragacanth, polyvinylpyrrolidine (Povidone), methylcellulose, ethylcellulose, sodium carboxymethylcellulose, hydroxypropylmethylcellulose, sucrose, and starch; fillers and carriers, for example corn starch, gelatin, lactose, sucrose, microcrystalline cellulose, kaolin, mannitol, dicalcium phosphate, sodium chloride, and alginic acid; disintegrators such as croscarmellose sodium, microcrystalline cellulose, corn starch, sodium starch glycolate, alginic acid and mutable wetting agents such as sodium lauryl sulfate; and lubricants such as magnesium stearate and other metallic stearates, stearic acid, silicone fluid, talc, waxes, oils and colloidal silica. Flavoring agents such as peppermint, oil of wintergreen, cherry flavoring or the like can also be used. It may be desirable to add a coloring agent to make the dosage form more aesthetically pleasing or to help identify the product. The tablets may also be coated by methods well known in the art.

The pharmaceutical compositions of the present invention may also be in the form of oral liquid preparations, which may be either a) aqueous or oily suspensions, solutions, emulsions or syrups, or b) a dry powder to be reconstituted with water or another suitable vehicle before use. When used in conjunction with such oral liquid preparations, the term "suitable vehicle" means conventional additives such as suspending agents such as acacia, methyl cellulose, hydroxyethyl cellulose, carboxymethyl cellulose, cellulose with sodium carboxymethyl cellulose (Avicel®), xantham gum, or starch; sweeteners such as sucrose, syrup, glucose, saccharin, sorbital, or aspartame; wetting agents such as sodium lauryl sulfate, silicone oil, the various Pluronics® surfactants, or glycerin; preservatives such as methyl, propyl, or butyl p-hydroxybenzoates, or sorbic acid; dyes, flavors and salts such as sodium chloride, citric acid, oil of wintergreen or sodium citrate; and waters, oils, and esters such as almond oil, fractionated coconut oil, hydrogenated caster oil, lecithin, aluminum stearate, and the like.

The crystalline dihydrate and trihydrate of LY163892 can also be formulated in unit dosage form in sterile vials, sterile plastic pouches containing a port with a septum, or sterile, hermetically sealed ampoules. The amount of the crystalline hydrate compound of the invention per unit dosage may vary from about 100 milligrams to about 10 grams. A preferred amount of compound will be in the range of from about 200 to 500 milligrams per unit dosage.

Topical compositions can be formulated with "suitable vehicles" such as hydrophobic or hydrophilic bases. Such bases include ointments, creams or lotions.

Veterinary pharmaceutical compositions of the antibiotic compounds may be administered in the feed or the drinking water of farm animals. Alternatively, the compounds can be formulated as intramammary preparations with "suitable vehicles" such as long- or quick-release bases.

A "therapeutically effective amount" of the instant hydrates of the antibiotic compound of Formula I is from approximately 2.5 mg to about 70 mg of compound per kilogram of body weight per dose. This amount generally totals from about 200 milligrams to about 7 grams per day for an adult human.

During a course of treatment with the instant hydrates, the hydrate of choice can be administered in a single daily dose or in multiple doses per day. The treatment regime may require administration over extended periods of time, for example, for several days or for from two to three weeks. The amount administered per dose or the total amount administered will depend on such factors as the nature and severity of the infection, the age and general health of the patient, and the tolerance of both the patient and the microorganism or microorganisms involved in the infection to the hydrates of Formula I.

As a further aspect of the present invention, there is provided a a process for preparing a crystalline dihydrate form by the compound of Formula (I):

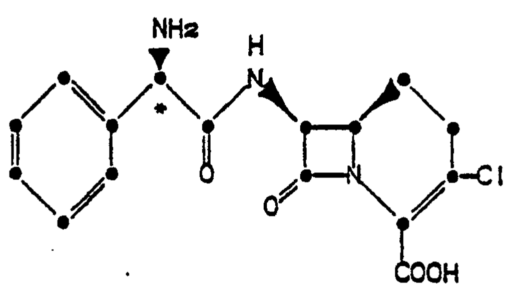

I

which comprises dissolving a mono or bis N,N′-dimethylformamide solvate of a compound of Formula (I) in water. and adjusting the pH to about 3.6, followed by filtration and air drying at room temperature.

As a further aspect of the present invention, there is provided a process for preparing a crystalline trihydrate form of the compound of Formula (I):

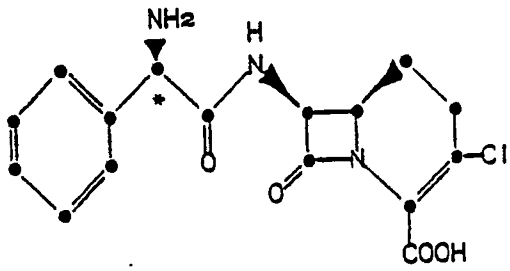

I

which comprises dissolving a mono or bis N,N′-dimethylformamide solvate of a compound of Formula (I) in water, adjusting the pH to about 4.8 to about 4.9, removing the monohydrate thus formed, and adding acetonitrile followed by filtration and air drying to provide said trihydrate.

In the following Examples, the terms dimethylformamide, nuclear magnetic resonance spectra, mass spectrum, infrared spectroscopy are abbreviated DMF, NMR, MS, and IR, respectively.

In conjunction with the NMR spectra, the following abbreviations are used: "s" is singlet, "d" is doublet, "t" is triplet, "q" is quartet, and "m" is multiplet.

The NMR spectra were obtained on a General Electric QE-300 300 MHz instrument unless otherwise stated. The chemical shifts are expressed in ppm values (parts per million downfield from tetramethylsilane).

## Experimental Section

### Procedure 1

Synthesis of LY163892 Mono(DMF)

To a solution of 30 g (0.126 mol) of 7β-amino-3-chloro-3-(1-carba-1-dethiacephem)-4-carboxylic acid in 500 ml of DMF containing 0.03% water was added approximately 10 drops of trimethylsilyl chloride followed by 32.85 g of N,N′-bis(trimethylsilyl)urea. The reaction mixture was stirred for 90 minutes at room temperature and cooled to approximately -50° C. To the mixture was added 10.8 ml of pyridine followed by 27.07 g (0.1315 mol) of 2-(R)-2-phenyl-2-aminoacetyl chloride hydrochloride. The mixture was stirred at about -35° C for approximately one hour and cooled to -50° C. To the mixture was added 7.32 ml of methanol in 30 ml of DMF. The reaction mixture was stirred to 0° C over a period of 40 minutes and 54 ml of water was added. The reaction mixture was allowed to warm to about 15° C over the next 30 minutes and triethylamine was slowly added until the pH of the mixture was about 3.2. The filtrate was warmed to about 50° C. To the mixture was added triethylamine until the pH reached 4.6 and the mixture was allowed to stand at room temperature for one hour. The mixture was then stirred at about 40° C and a mixture of DMF: triethylamine (1:1,v:v) was added until the pH was about 5.9. The mixture was cooled to 25° C and stirred for 20 minutes. To the mixture was added 500 ml of acetonitrile and the resulting mixture was stirred for 30 minutes. The mixture was filtered and the precipitated solid was dried in an air oven at 30° C until the weight of the solid was constant to provide LY163892 mono(DMF).

H-NMR (300 MHz, $D_2O$/DCl): 8.18 ppm (s,1H); 7.79 (s,1H); 5.65 (d, J=4.8 Hz, 1H); 5.51 (s,1H); 4.21 (d of t, 1H); 3.28 (s, 3H); 3.12 (s, 3H); 2.84 (m, 2H); 1.95 (m, 1H); 1.58 (m, 1H). $^{13}C$-NMR (75.48 MHz $D_2O$/DCl): 21.99, 31.85, 32.27, 37.77, 53.47, 57.37, 58.57, 123.5, 128.9, 130.6, 131.4 132.5, 133.8, 164.4 165.7, 166.3, 169.9 ppm.

IR (KBr disc): 2950-3620 $cm^{-1}$ (m and broad) 1772.7, 1691.7, 1658.9, 1598, 1566, 1409, 1389, 1378, 1349, 1325 (all medium to strong).

$[\alpha]_D^{20}$ = +17.85°, C = 1.02 in 0.1 N HCl.
MS = 350, 352

Procedure 2

Synthesis of LY163892 Bis(DMF)

Powdered sodium 2-(R)-2-phenyl-2-(((Z)-methyl but-2-en-3-yloate)amino)acetate (4.59 g, 16.92 mmol) was sifted into a 250 ml round bottom flask containing 75 ml of stirred DMF under a nitrogen atmosphere. Next 22 µl of methanesulfonic acid was added to the reaction mixture which was cooled to about -45° C. To the mixture was added 47.6 µl of dimethylbenzylamine followed by 1.53 g (1.25 ml, 16.15 mmol) of methyl chloroformate. The reaction mixture was stirred at about -45° C for approximately 50 minutes. To the mixture was added a solution of 25 ml of DMF and 5.41 g (15.38 mmol) of 7β-amino-3-chloro-3-(1-carba-1-dethiacephem)-4-carboxylic acid (4-nitrophenyl)methyl ester dropwise to the reaction mixture. The mixture was stirred for two hours and warmed to about 0° C for approximately 45 minutes. While maintaining the temperature of the reaction mixture between about 5° C and 10° C, 6.89 ml of water, 12.32 ml of concentrated hydrochloric acid, 3.55 g of zinc dust and 8.93 ml of concentrated hydrochloric acid were added to the reaction mixture. The mixture was stirred at room temperature for about five hours and the pH was adjusted to 2.35 with triethylamine. The mixture was filtered and the pH was again adjusted to 4.6 with triethylamine. The mixture was stirred for 45 minutes and the pH was raised to 5.75 with triethylamine. The mixture was stirred for an additional 15 minutes. The precipitated solid was collected by vacuum filtration and washed with 30 ml of DMF:water (9:1, v:v). The solid was vacuum dried for approximately 10 hours to provide 4.8 g of the bis(DMF) solvate of LY163892.

This material was further purified by suspending the material in 40 ml of DMF:water (9:1, v:v). The solution was cooled to 10° C and the pH was adjusted to 1.7 with concentrated hydrochloric acid. The mixture was filtered and the pH was raised to 5.6 with triethylamine. The precipitated solid was collected by vacuum filtration, washed with DMF:water (9:1, v:v) and vacuum dried at 26° C to provide 3.72 g of bis-(DMF) LY163892.
H-NMR (300 MHz, $D_2O$/DCl): 8.18 ppm (s, 2H); 7.79 (s,1H); 5.65 (d, 1H); 5.51 (s, 1H); 4.21 (d of t, 1H); 3.28 (s, 6H); 3.12 (s, 6H); 2.84 (m, 2H); 1.95 (m, 1H); 1.58 (m, 1H).
IR (KBr disc): 1772.7 $cm^{-1}$, 1691.7, 1659.9, 1599.1, 1566.3, 1407.2, 1389.8, 1378.2, 1349.3, 1325.2 (all medium to strong)
$[\alpha]_D^{20}$ = +12.68°, c = 0.35 in 0.01 N HCl.
MS = 350, 352

Example 1

LY163892 Dihydrate

LY163892 mono(DMF) solvate (79.83 g) was suspended in water (435 ml). To the suspension was added concentrated hydrochloric acid (54.5 ml) and sodium EDTA (2.18 g). The suspension was stirred for fifteen minutes at room temperature to give a solution. Charcoal (Darco®, 2.18 g) was added to the solution and the resultant suspension was filtered with the help of Hyflo® (celite) filter aid. The filter cake was washed with distilled water (200 ml). The pH of the combined filtrate and wash was adjusted to 2.4 by the addition of triethylamine, and the solution was seeded with crystals of LY163892 dihydrate. The resultant suspension was stirred for thirty minutes. The pH of the suspension was then taken to 3.6 over a forty-five minute period by the addition of triethylamine. The suspension was stirred for five hours, cooled to 10° C, then filtered. The filter cake was allowed to dry in the open at room temperature. The dried filter cake was stirred with water (300 ml) and filtered. The filter cake was washed with acetonitrile (400 ml). The filter cake was allowed to dry in the open at room temperature, yielding 26.63 g of crystalline LY163892 dihydrate: NMR (300 MHz, DCl/$D_2O$): ppm 7.42 (5H,s), 5.25 (1H,d), 4.74 (1H,s), 3.81 (1H,d of t), 2.44 (2H, m), 1.57

(1H,m), 1.22 (1H, d of d); X-ray: (the X-ray powder diffraction parameters and pattern for the compound produced in this Example are provided in Table I above).

## Example 2

### LY163892 Trihydrate

#### Procedure A

LY163892 bis(DMF) solvate (4.0 g) was suspended in water (30.8 ml) and the suspension was heated to 50° C. The pH of the suspension was adjusted to 1.57 by the addition of concentrated hydrochloric acid. To the suspension was added sodium EDTA (0.05 g). The suspension was stirred for thirty minutes and charcoal (Darco®) was added and the suspension was filtered. The filtrate was seeded with crystals of LY163892 monohydrate and the pH of the suspension was adjusted to 2.08 by the addition of concentrated hydrochloric acid to induce crystallization. The suspension was stirred for thirty minutes, at the end of which time the pH was adjusted from 1.9 to 4.8 by the addition of triethylamine. The suspension was cooled to room temperature, filtered, and the collected solid (crude monohydrate) was washed with water (6 ml). The filtrate was mixed with an equal volume of acetonitrile. The resultant mixture was stirred at room temperature for one hour and filtered. The filter cake was washed with acetonitrile and allowed to dry in the open at room temperature to yield 0.48 g of crystalline LY163892 trihydrate. Karl Fischer analysis: 14.4% moisture.

#### Procedure B

LY163892 bis(DMF) solvate (1.30 g) was suspended in water (10 ml). The suspension was warmed to 50° C. Sodium EDTA (0.01 g) was added to the warmed suspension. The pH of the suspension was adjusted to 8.6 by the addition of saturated aqueous sodium bicarbonate solution, resulting in a solution. The solution was stirred for twenty minutes. The pH of the solution was slowly lowered to 7.6 by the addition of hydrochloric acid (2N). The solution was seeded with crystals of LY163892 monohydrate. The pH of the solution was again adjusted by the addition of aqueous hydrochloric acid to 7.0 to induce precipitation. The suspension was stirred for thirty minutes. The pH of the suspension was again adjusted to about 7.0 (from 7.7) by the addition of aqueous hydrochloric acid. After the pH of the suspension stabilized at 7.2, the pH was lowered to approximately 4.6 by the addition of concentrated hydrochloric acid. The solution was stirred for thirty minutes to make sure the pH of the solution was stable and cooled to room temperature and stirred for two hours. The suspension was filtered and the collected crystals washed with water (approx. 4 ml). The crystals were allowed to air dry at room temperature to give 0.59 g, 56.7% yield of crystalline LY163982 trihydrate. Karl Fischer analysis: 11.3% moisture content.

## Example 3

### LY163892 Monohydrate from LY163892 Trihydrate

The following Procedure was carried out according to the standards of the Food and Drug Administrations Good Manufacturing Practices. Water (1.62 L) and hydrochloric acid (12 molar, 43.0 ml) were combined and stirred at 20° C. 7β-[2′(R)-2′-phenyl-2′-aminoacetamido]-3-chloro-3-(1-carba-1-dethiacephem)-4-carboxylic acid trihydrate (200.0 g) was added to the solution and the resultant suspension

was stirred for 30 minutes. Additional hydrochloric acid (10 ml) was added and the suspension was stirred for 20 minutes to effect solution. Charcoal (8.0 g) was added to the solution and the resultant suspension was stirred for 30 minutes. The suspension was filtered through a HYFLO® filter aid pad on glass fiber paper (the filter was then washed with 100 ml of water). The filtrate was warmed to 45° C over a 25 minute period. Triethylamine was slowly added to the filtrate to raise the pH of the solution to 1.8 (the temperature of the filtrate was maintained between 45° and 50° C during the addition). The solution was seeded with 100 mg of LY163892 monohydrate, resulting in a suspension. The suspension was stirred for 1 hour at 50° C. The pH of the suspension was raised to 4.8 slowly by the addition of triethylamine. The suspension was stirred for an additional 15 minutes at 50° C then cooled to 25° C and stirred for I hour. The suspension was filtered on a 24 cm Büchner funnel fitted with a polypropylene pad. The filter cake was washed with water (2X, 250 ml), then the funnel was covered and vacuum was applied to the filter cake overnight. The filter cake was put in a cleaned air dryer at 30° C for 24 hours to give 119.90 g, 65.9% yield of crystalline LY163892 monohydrate.

## Example 4

## LY163892 Monohydrate from LY163892 Dihydrate

LY163892 Dihydrate (1.1 g) was suspended in 9.5 ml of water at a temperature of about 45° C. Next, 2N sodium hydroxide was added until a solution resulted (at about pH 7.6). The solution was filtered and 2N hydrochloric acid was added until the pH was approximately 4. The mixture was filtered and the collected solid was washed with hot water. The crystals were air dried to provide LY163892 monohydrate as compared by X-ray crystallography to an authentic reference standard. Karl Fischer analysis = 5.78% moisture.

In the following Examples 5 through 9 the term "LY163892 active ingredient" is the dihydrate or trihydrate, or a mixture of one or more of these hydrates. The term preferably indicates the microcrystalline hydrate with the X-ray powder diffraction patterns of Tables I and II above, and especially the dihydrate with the pattern of Table I.

## Example 5

Hard gelatin capsules are prepared using the following ingredients:

| | Quantity (mg/capsule) |
|---|---|
| LY163892 active ingredient | 200 |
| starch flowable powder | 186 |
| starch flowable powder with silicone 5% | 50 |
| magnesium stearate | 2.5 |

The above ingredients are mixed and filled into hard gelatin capsules in 438.5 mg quantities.

## Example 6

A tablet formula is prepared using the ingredients below:

| | Quantity (mg/tablet) |
|---|---|
| LY163892 active ingredient | 200 |
| cellulose, microcrystalline | 200 |
| silicon dioxide, fumed | 10 |
| stearic acid | 5 |

The components are blended and compressed to form tablets each weighing 415 mg.

Example 7

Suppositories each containing 200 mg of active ingredient are made as follcws:

| | |
|---|---|
| LY163892 active ingredient | 200 mg |
| saturated fatty acid glycerides to | 2000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

| Example 8 | |
|---|---|
| Ready-To-Use Aqueous Suspension | |
| LY163892 active ingredient | 210 mg |
| cellulose with sodium carboxymethylcellulose | 95 mg |
| sucrose | 1.85 g |
| parafens | 3 mg |
| emulsion silicone | 2.5 mg |
| pluronic | 5 mg |
| flavor | 2 mg |
| color | 0.5 mg |
| purified water | q.s. to 5 ml |

The ingredients are sieved. The parafens are dissolved in hot purified water and, after cooling, the other ingredients are added. Sufficient purified water is added to produce the required volume. The suspension can then be passed through a colloid mill or homogenizer to produce a more elegant dispersion.

| Example 9 | |
|---|---|
| Suspension for Reconstitution | |
| LY163892 active ingredient | 210 mg |
| sucrose | 3 g |
| xanthan gum | 5 mg |
| starch modified | 10 mg |
| emulsion silicone | 5 mg |
| sodium lauryl sulfate | 0.5 mg |
| methylcellulose | 2 mg |
| flavor | 0.5 mg |
| dye | |

The ingredients are sieved and mixed in a suitable blender, such as a twin-shell, twin core Nauta®-type, or ribbon blender. To constitute, a sufficient volume of purified water is added to produce the required volume.

## Claims

1. A crystalline dihydrate form of the compound of the Formula I

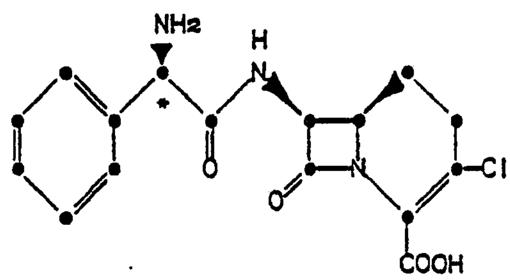

I

2. A compound of Claim 1, which has the following X-ray powder diffraction pattern obtained with a nickel-filtered copper radiation of λ = 1.5418 Å wherein d represents the interplanar spacing and $I/I_1$ the relative intensity:

| d | I/I1 |
|---|---|
| 16.35 | 1.00 |
| 9.82 | .05 |
| 9.50 | .05 |
| 8.18 | .90 |
| 6.46 | .01 |
| 6.32 | .04 |
| 5.40 | .02 |
| 4.72 | .11 |
| 4.48 | .04 |
| 4.07 | .19 |
| 3.88 | .02 |
| 3.78 | .07 |
| 3.60 | .08 |
| 3.45 | .04 |
| 3.31 | .01 |
| 3.24 | .05 |
| 3.10 | .02 |
| 2.99 | .05 |
| 2.92 | .05 |

3. A pharmaceutical composition for the treatment of bacterial infections in warm-blooded animals, which comprises a therapeutically-effective amount of the compound of Claim 1 and a suitable vehicle.

4. A pharmaceutical composition for the treatment of bacterial infections in warm-blooded animals, which comprises a therapeutically-effective amount of the compound of Claim 2 and a suitable vehicle.

5. A crystalline trihydrate form of the compound of the Formula I

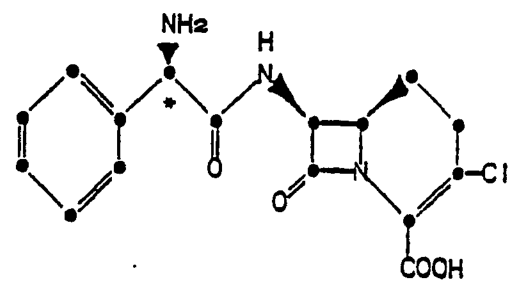

I

6. A compound of Claim 5, which has the following X-ray powder diffraction pattern obtained with a nickel-filtered cooper radiation of $\lambda$ = 1.5418 Å wherein d represents the interplanar spacing and $I/I_1$ the relative intensity:

| d | I/I1 |
|---|---|
| 11.94 | 1.00 |
| 7.49 | .15 |
| 5.94 | .05 |
| 5.27 | .03 |
| 4.67 | .02 |
| 3.95 | .18 |
| 3.63 | .03 |
| 3.34 | .03 |
| 3.07 | .03 |

7. A pharmaceutical composition for the treatment of bacterial infections in warm-blooded animals,

which comprises a therapeutically-effective amount of the compound of Claim 5 and a suitable vehicle.

8. A pharmaceutical composition for the treatment of bacterial infections in warm-blooded animals, which comprises a therapeutically-effective amount of the compound of Claim 6 and a suitable vehicle.

9. A crystalline dihydrate or trihydrate of a compound of Formula (I) as claimed in any one of Claims 1-6 for use as an antibacterial agent.

10. A process for preparing a crystalline dihydrate form of the compound of Formula (I):

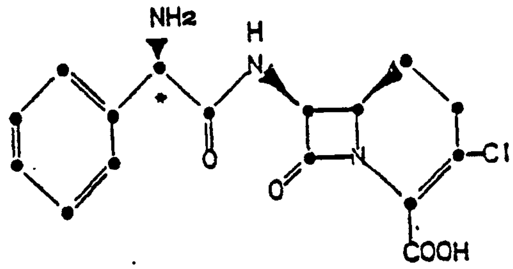
I

which comprises dissolving a mono or bis N,N′-dimethylformamide solvate of a compound of Formula (I) in water, and adjusting the pH to about 3.6, followed by filtration and air drying at room temperature.

11. A process for preparing a crystalline trihydrate form of the compound of Formula (I):

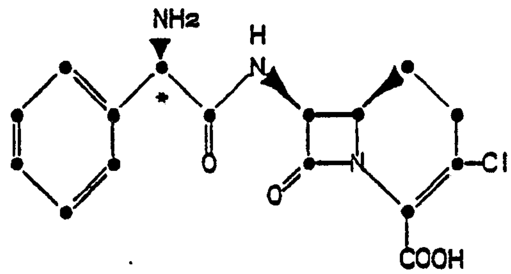
I

which comprises dissolving a mono or bis N,N′-dimethylformamide solvate of a compound of Formula (I) in water, adjusting the pH to about 4.8 to about 4.9, removing the monohydrate thus formed, and adding acetonitrile followed by filtration and air drying to provide said trihydrate.

Claims for the following Contracting States: GR, ES

1. A process for preparing a crystalline dihydrate form of the compound of Formula (I):

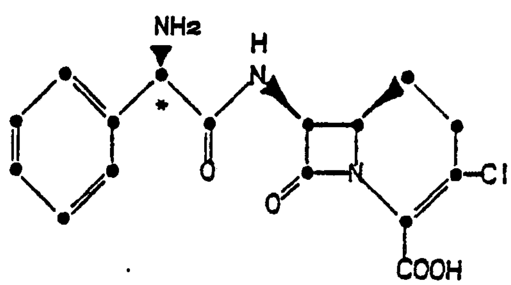
I

which comprises dissolving a mono or bis N,N′-dimethylformamide solvate of a compound of Formula (I) in water, and adjusting the pH to about 3.6, followed by filtration and air drying at room temperature.

2. A process for preparing a crystalline dihydrate of a compound of Formula I according to Claim 1, which has the following X-ray powder diffraction pattern obtained with a nickel-filtered copper radiation of λ = 1.5418 Å wherein d represents the interplanar spacing and $I/I_1$ the relative intensity:

| d | $I/I_1$ |
|---|---|
| 16.35 | 1.00 |
| 9.82 | .05 |
| 9.50 | .05 |
| 8.18 | .90 |
| 6.46 | .01 |
| 6.32 | .04 |
| 5.40 | .02 |
| 4.72 | .11 |
| 4.48 | .04 |
| 4.07 | .19 |
| 3.88 | .02 |
| 3.78 | .07 |
| 3.60 | .08 |
| 3.45 | .04 |
| 3.31 | .01 |
| 3.24 | .05 |
| 3.10 | .02 |
| 2.99 | .05 |
| 2.92 | .05 |

3. A process for preparing a crystalline trihydrate form of the compound of Formula (I):

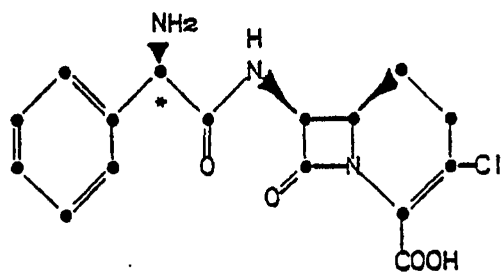

I

which comprises dissolving a mono or bis N,N′-dimethylformamide solvate of a compound of Formula (I) in water, adjusting the pH to about 4.8 to about 4.9, removing the monohydrate thus formed, and adding acetonitrile followed by filtration and air drying to provide said trihydrate.

4. A process for preparing a crystalline trihydrate of a compound of Formula (I) according to Claim 3, which has the following X-ray powder diffraction pattern obtained with a nickel-filtered cooper radiation of $\lambda$ = 1.5418 Å wherein d represents the interplanar spacing and $I/I_1$ the relative intensity:

| d | $I/I_1$ |
|---|---|
| 11.94 | 1.00 |
| 7.49 | .15 |
| 5.94 | .05 |
| 5.27 | .03 |
| 4.67 | .02 |
| 3.95 | .18 |
| 3.63 | .03 |
| 3.34 | .03 |
| 3.07 | .03 |

5. A method of preparing a pharmaceutical formulation which comprises admixing a dihydrate or trihydrate of a compound of Formula I

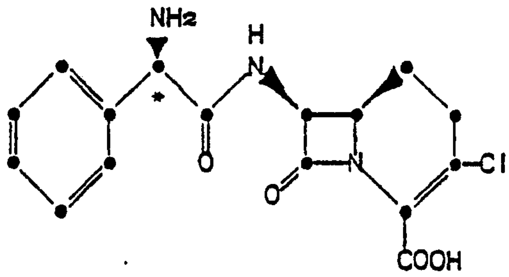

I

with one or more pharmaceutically-acceptable carriers, diluents or excipients therefor.

European Patent Office

# EUROPEAN SEARCH REPORT

Application Number

EP 89 31 1636

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 014 476 (KYOWA HAKKO KOGYO K.K.) * Pages 37-38, example 12; claims * & US-A-4 335 211 (Cat. D) | 1-9 | C 07 D 463/00<br>A 61 K 31/435 |
| P,Y | EP-A-0 327 364 (ELI LILLY) * Claims * | 1-11 | |
| D,P Y | EP-A-0 311 366 (ELI LILLY) * Claims * | 1-11 | |

TECHNICAL FIELDS SEARCHED (Int. Cl.5)

C 07 D 463/00
C 07 D 471/00
A 61 K 31/00

The present search report has been drawn up for all claims

| Place of search | Date of completion of the search | Examiner |
|---|---|---|
| THE HAGUE | 05-02-1990 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)